(19) Europäisches Patentamt
European Patent Office
Office européen des brevets

(11) EP 1 892 014 B1

(12) FASCICULE DE BREVET EUROPEEN

(45) Date de publication et mention de la délivrance du brevet:
**14.09.2011 Bulletin 2011/37**

(51) Int Cl.:
***A61Q 5/06*** *(2006.01)* ***A61K 8/04*** *(2006.01)*
***A61K 8/73*** *(2006.01)*

(21) Numéro de dépôt: **07113242.7**

(22) Date de dépôt: **26.07.2007**

(54) **Composition cosmétique comprenant au moins un polysaccharide de type carraghénane lambda sous forme d'aérosol ; procédé de traitement cosmétique des fibres kératiniques et utilisation de la composition**

Kosmetische Zusammensetzung in Aerosol Form enthaltend mindestens ein Polysaccharid vom Lambda-Carrageenan Typ; kosmetische Behandlungsmethode für Keratinfasern und Verwendung der Zusammensetzung

Cosmetic composition containing at least one lambda carrageenan type polysaccharide in aerosol form; cosmetic treatment method of keratin fibres and use of the composition

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **04.08.2006 FR 0607156**

(43) Date de publication de la demande:
**27.02.2008 Bulletin 2008/09**

(73) Titulaire: **L'Oréal**
**75008 Paris (FR)**

(72) Inventeurs:
- **Beitone, Régis**
**75645 Paris Cedex 13 (FR)**
- **Laurent, Ludivine**
**92400 Courbevoie (FR)**

(74) Mandataire: **Dossmann, Gérard**
**Casalonga & Partners**
**Bayerstrasse 71-73**
**80335 München (DE)**

(56) Documents cités:
**EP-A- 0 445 659** **EP-A- 0 664 113**
**EP-A2- 0 920 851** **US-A1- 2006 134 049**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 892 014 B1

## Description

La présente invention concerne une composition cosmétique pour le traitement des fibres kératiniques, en particulier des fibres kératiniques humaines telles que les cheveux, sous forme d'aérosol comprenant au moins un polysaccharide de type carraghénane lambda, du dioxyde de carbone en tant que gaz propulseur et au moins un additif spécifique, un procédé de traitement cosmétique la mettant en oeuvre ainsi qu'une utilisation de cette composition pour la fixation des fibres kératiniques.

Parmi les compositions cosmétiques pour la mise en forme et/ou le maintien de la coiffure les plus répandues sur le marché de la cosmétique, on trouve des compositions à pulvériser ou à disperser sous forme de mousse essentiellement constituées d'une solution le plus souvent alcoolique ou hydroalcoolique et d'un ou plusieurs composants, généralement des résines polymères, dont la fonction est soit de former des soudures entre les cheveux, soit de gainer ceux-ci. Ces composants sont généralement appelés composants fixants, et sont souvent en mélange avec divers adjuvants cosmétiques. Cette solution est généralement conditionnée soit dans un récipient aérosol approprié mis sous pression à l'aide d'un agent propulseur, soit dans un flacon pompe.

On connaît de nombreux systèmes aérosols destinés à fixer ou à gainer les chevelures, ces systèmes contenant d'une part une phase liquide (ou jus) et d'autre part un agent propulseur. Ce dernier a pour fonction d'assurer une pression permettant de disperser la phase liquide sous forme d'un spray ou d'une mousse.

Récemment, les carraghénanes ont été utilisés en tant que polymères fixants dans des gels de coiffage ou dans des aérosols. En effet, il est connu du document EP 1 199 064 de mettre en oeuvre un carraghénane ou un mélange de ceux-ci et un additif particulier pour obtenir un gel solide et stable pour le traitement capillaire.

Les carraghénanes sont des polysaccharides qui constituent les parois cellulaires de diverses algues rouges (Rhodophycées) appartenant aux familles des *Gigartinaceae, Hypneaceae, Furcellariaceae* et *Polyideaceae.* Ils comportent des longues chaînes galactanes, polyélectrolytes anioniques. Leur masse moléculaire peut être supérieure à $10^6$. Ces polymères linéaires, formés par des motifs disaccharides, sont composés par deux unités D-galactopyranoses liées alternativement par des liaisons α- et β-. Ce sont des polysaccharides très sulfatés (20-50%) et les résidus α-D-galactopyranosyles peuvent être sous forme 3',6'-anhydro.

Initialement, les carraghénanes ont été subdivisés en deux familles suivant leur solubilité dans le chlorure de potassium (KCl). Les fractions solubles dans le KCl ont été désignées par les préfixes "Kappa", tandis que les termes "Lambda" ont été réservés à celles insolubles. Plus tard, les classifications ont été basées sur le nombre, la position de groupements sulfates ainsi que la présence de pont 3',6'-anhydro sur les résidus β-D-galactopyranosyles. Ceci a abouti aux quatre grandes familles :κ, λ, β,ω.

Les différents types de carraghénanes n'existent pas à l'état pur, mais sous forme d'hybrides. Ainsi à l'état naturel, les κ et ι-carraghénanes se présentent sous une forme hybride Kappa-iota mais l'une des deux structures peut prédominer sur l'autre. L'état hybride κ-ι d'une structure peut être élucidé en utilisant des enzymes spécifiques, qui permettent d'enrichir ou de diminuer la teneur en l'une des deux formes. Les carraghénanes peuvent coexister avec leurs précurseurs. Les carraghénanes de différentes familles d'appartenance peuvent coexister dans une structure hybride. Ex.: carraghénane de Euchema gelatinae : une hybride de β-carraghénane, composant majeur, et de κ, γ-carraghénanes.

Les compositions capillaires sous forme d'aérosol contenant un polysaccharide de type λ-carraghénane permettent d'obtenir des mousses capillaires avec de très bonnes propriétés de coiffage et de soin. Toutefois, l'utilisation de gaz propulseurs classiques, de type hydrocarbures et mélanges d'hydrocarbures, ne permet pas d'obtenir des mousses satisfaisante.

Il existe donc un réel besoin de trouver des compositions cosmétiques, notamment pour le coiffage, qui permettent d'améliorer la qualité de la mousse et qui permettent d'obtenir des gels de meilleures textures, moins cassants, moins durs et plus faciles à appliquer.

De manière surprenante et avantageuse, la Demanderesse vient de découvrir qu'en utilisant du dioxyde de carbone, en tant que gaz propulseur, pour des composition à base d'au moins un polysaccharide de type λ-carraghénane, il était possible d'obtenir une mousse coiffante de texture originale : mousse crémeuse, glissante et peu expansée.

D'autre part, l'utilisation de dioxyde de carbone en tant que gaz propulseur en association avec un polysaccharide de type λ-carraghénane permet d'obtenir une mousse entièrement naturelle.

La présente invention a donc notamment pour objet une composition cosmétique pour le traitement des fibres kératiniques, en particulier des fibres kératiniques humaines telles que les cheveux, comprenant, dans un milieu cosmétiquement acceptable :

- au moins un polysaccharide de type carraghénane lambda,
- du dioxyde de carbone en tant que gaz propulseur, et au moins un additif choisi parmi une silicone, un corps gras

et un polymère fixant différent du polysaccharide de type carraghénane lambda.

L'invention a également pour objet un procédé de traitement cosmétique mettant en oeuvre la composition cosmétique selon l'invention.
Un autre objet de la présente invention consiste en une utilisation de la composition cosmétique selon l'invention pour la fixation des cheveux ou pour le soin des cheveux.
D'autres objets, caractéristiques, aspects et avantages de l'invention apparaîtront encore plus clairement à la lecture de la description et des exemples qui suivent.
Par le mot « coiffage », on entend le fait de fixer et/ou de maintenir la forme de la coiffure.
La composition selon l'invention comprend au moins un polysaccharide de type carraghénane lambda.
De préférence, le polysaccharide de type carraghénane lambda utilisé selon la présente invention n'est pas modifié chimiquement.
De préférence, le poids moléculaire (PM) du polysaccharide est compris entre 100 000 et 1 000 000. Encore plus préférentiellement, le poids moléculaire est compris entre 250 000 et 800 000.
A titre de polysaccharide de type carraghénane lambda utilisable dans le cadre de la présente invention, on peut citer le SATIAGUM UTC 10 de la société DEGUSSA et le WELGEENAN ED 1039 de la société EUROGUM.
Le polysaccharide de type carraghénane lambda peut être présent dans la composition à une teneur comprise entre 0,1 et 30 % de préférence entre 0,2 et 20 %, et encore plus préférentiellement entre 0,5 et 15 % en poids du poids total de la composition aérosol.
Le moyen de distribution est généralement constitué par une valve de distribution commandée par une tête de distribution, elle même comprenant une buse par laquelle la composition aérosol est vaporisée.
Selon un mode préférentiel de réalisation de l'invention, le dispositif aérosol selon l'invention est approprié pour obtenir un débit en matière sèche supérieur ou égal à 20 mg/s, de préférence compris entre 20 mg/s et 60 mg/s.
Selon la présente invention, le débit en matière (DMS) sèche correspond à la quantité d'extrait sec qui sort du dispositif aérosol par unité de temps. Ce débit en matière sèche est exprimé en mg/s et est calculé en multipliant la concentration en matière sèche dans la composition aérosol (CMS) par le débit de la composition aérosol à la sortie de la buse (DCA):

$$DMS = CMS \times DCA.$$

La concentration en matière sèche dans la composition aérosol (CMS) correspond à la quantité de matière sèche ramenée à 100g de composition aérosol (jus + propulseur). La concentration en matière sèche est exprimée en pourcentage et est mesurée après pulvérisation par évaporation des composants volatils du résidu de pulvérisation pendant 1 heure 30 à 105 °C.
Le débit en composition aérosol (DCA) correspond à la quantité de composition aérosol (jus + propulseur) sortant du dispositif aérosol par unité de temps. Il est exprimé en mg/s et est mesuré par la différence entre le poids de l'aérosol avant (M0) et après (M1) 10 secondes de vaporisation:

$$DCA = (M0 - M1) / 10.$$

D'une manière avantageuse, la concentration en matière sèche (CMS) est comprise entre 2,5 et 15 % en poids par rapport au poids total de la composition aérosol (jus + propulseur), de préférence comprise entre 3,5 et 10 % en poids.
Le débit en composition aérosol (DCA) sera alors approprié pour obtenir un débit en matière sèche (DMS) tel que défini ci-dessus. De manière préférentielle, le DCA sera compris entre 500 et 800 mg/s, plus préférentiellement voisin de 600 mg/s.
La phase de la composition aérosol est de préférence une phase longue, c'est à dire que le rapport pondéral jus/propulseur est supérieur à 1, plus préférentiellement compris entre 1,2 et 3.
De préférence, l'agent propulseur représente 2 à 70%, de préférence 3 à 50% en poids par rapport au poids total de l'ensemble des compositions contenues dans le dispositif aérosol.
En fonction de la composition aérosol (jus + propulseur), l'homme du métier saura sélectionner le moyen de distribution approprié pour obtenir les caractéristiques de débit en matière sèche souhaité.
Les caractéristiques particulières définies ci-dessus (CMS et phase) peuvent être obtenues en sélectionnant les moyens de distribution appropriés et/ou en agissant sur la formulation.
Les valves appropriées pour les compositions particulières ci-dessus sont notamment des valves droites avec

un gicleur de diamètre compris entre 0,35 et 0,60 mm, de préférence compris entre 0,40 et 0,50 mm, avantageusement sans restriction interne ni prise de gaz additionnelle. Il s'agit en particulier des valves commercialisées sous la dénomination COSTER T104 RA36/0/4 par la société COSTER, ou de la valve Précision Expérimentale 15130 constituée par un gicleur et un corps de valve de 0,46 mm de diamètres sans prise de gaz additionnelle de la société Précision.

Les diffuseurs appropriés pour les compositions particulières ci-dessus sont en particulier les boutons poussoirs commercialisés sous la dénomination Précision 216903-50AD29 par la société Précision.

La composition selon l'invention comprend au moins un additif choisi parmi une silicone, un corps gras et un polymère fixant différent du polysaccharide de type carraghénane lambda.

La composition cosmétique selon l'invention peut comprendre en outre au moins une silicone.

Dans tout ce qui suit, on entend désigner par silicone, en conformité avec l'acceptation générale, tous polymères ou oligomères organosiliciés à structure linéaire ou cyclique, ramifiée ou réticulée, de poids moléculaire variable, obtenus par polymérisation et/ou par polycondensation de silanes convenablement fonctionnalisés, et constitués pour l'essentiel par une répétition de motifs principaux dans lesquels les atomes de silicium sont reliés entre eux par des atomes d'oxygène (liaison siloxane -Si-O-Si-), des radicaux hydrocarbonés éventuellement substitués, étant directement liés par l'intermédiaire d'un atome de carbone sur lesdits atomes de silicium. Les radicaux hydrocarbonés les plus courants sont les radicaux alkyles, notamment en $C_1$-$C_{10}$, et en particulier méthyle, les radicaux fluoroalkyles dont la partie alkyle est en $C_1$-$C_{10}$, les radicaux aryles et en particulier phényle.

De préférence, la silicone est une silicone oxyalkylénée.

Par silicone oxyalkylénée, on entend toute silicone comportant au moins un groupement oxyalkyléné de type $(-C_xH_{2x}O-)_a$ dans lequel x peut varier de 2 à 6, et a est supérieur ou égal à 2.

Les silicones oxyalkylénées utilisables dans la composition cosmétique sont choisies parmi les formules générales (VI), (VII), (VIII) ou (IX) suivantes:

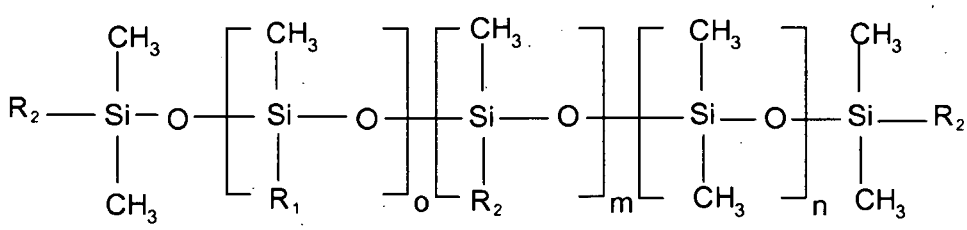

(VI)

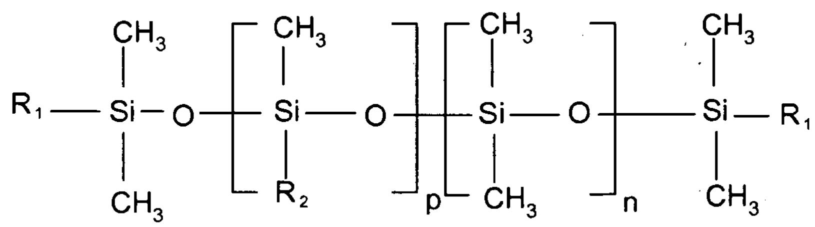

(VII)

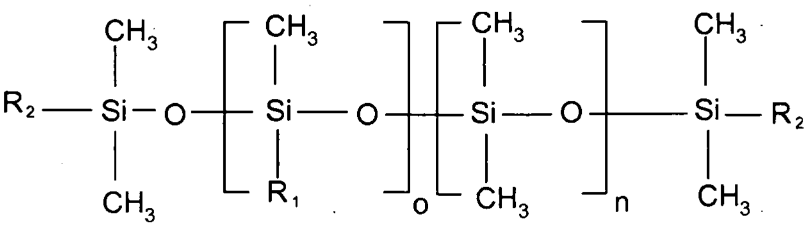

(VIII)

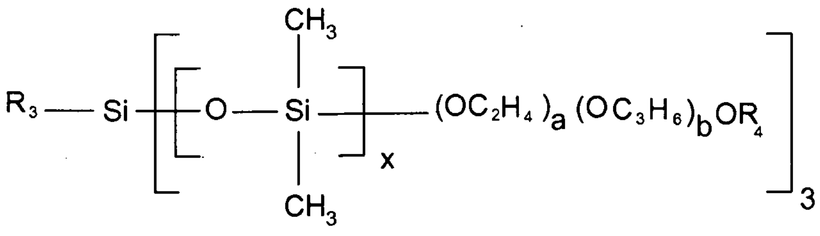

(IX)

dans lesquelles:

- $R_1$, identique ou différent, représente un radical alkyle, linéaire ou ramifié, en $C_1$-$C_{30}$ ou phényle,
- $R_2$, identique ou différent, représente un radical $-C_cH_{2c}-O-(C_2H_4O)_a(C_3H_6O)_b-R_5$ ou un radical $-C_cH_{2c}-O-(C_4H_80)_a-R_5$,
- $R_3$, $R_4$, identiques ou différents, désignent un radical alkyle, linéaire ou ramifié, en $C_1$ à $C_{12}$, et de préférence le radical méthyle,
- $R_5$, identique ou différent, est choisi parmi un atome d'hydrogène, un radical alkyle, linéaire ou ramifié, comprenant de 1 à 12 atomes de carbone, un radical alcoxy, linéaire ou ramifié, comprenant de 1 à 6 atomes de carbone, un radical acyle, linéaire ou ramifié, comprenant de 2 à 30 atomes de carbone, un radical hydroxyle, $-SO_3M$, aminoalcoxy en $C_1$-$C_6$ éventuellement substitué sur l'amine, aminoacyle en $C_2$-$C_6$ éventuellement substitué sur l'amine, $-NHCH_2CH_2COOM$, - $N(CH_2CH_2COOM)_2$, aminoalkyle éventuellement substitué sur l'amine et sur la chaîne alkyle, carboxyacyle en $C_2$-$C_{30}$, un groupement éventuellement substitué par un ou deux radicaux aminoalkyle substitués, $-CO(CH_2)_dCOOM$, $-COCHR_7(CH_2)_dCOOM$, - $NHCO(CH_2)_dOH$, $-NH_3Y$, un groupement phosphate,
- M, identique ou différent, désigne un atome d'hydrogène, Na, K, Li, $NH_4$ ou une amine organique,
- $R_7$ désigne un atome d'hydrogène ou un radical $SO_3M$,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 2,
- c varie de 0 à 4,
- x varie de 1 à 100,
- Y représente un anion minéral ou organique monovalent tel que halogénure (chlorure, bromure), sulfate, carboxylate (acétate, lactate, citrate),

sous réserve que lorsque la silicone est de formule (VII) avec $R_5$ désignant hydrogène alors n est supérieur à 12.

De telles silicones sont par exemple commercialisées par la société GOLDSCHMIDT sous les dénominations commerciales ABIL WE 09, ABIL EM 90, ABIL B8852, ABIL B8851, ABIL B 8843, ABIL B8842, par la société DOW CORNING sous les dénominations FLUID DC 190, DC 3225 C, Q2-5220, Q25354, Q2-5200, par la société RHODIA CHIMIE sous les dénominations SILBIONE HUILE 70646, RHODORSIL HUILE 10634, par la société GENERAL ELECTRIC sous les dénominations SF1066, SF1188, par la société SWS SILICONES sous la dénomination SILICONE COPOLYMER F 754, par la société AMERCHOL sous la dénomination SILSOFT BEAUTY AID SL, par la société SHIN-ETSU sous la dénomination KF 351, par la société WACKER sous la dénomination BELSIL DMC 6038, par la société SILTECH sous les dénominations SILWAX WD-C, SILWAX WD-B, SILWAX WD-IS, SILWAX WSL, SILWAX DCA 100, SILTECH AMINE 65, par la société FANNING CORPORATION sous les dénominations FANCORSIL SLA, FANCORSIL LIM1, par la société PHOENIX sous la dénomination PECOSIL.

Ces silicones sont notamment décrites dans les brevets US-A-5 070 171, US-A-5149765, US-A-5093452 et US-A-5091493.

De préférence, on utilise les silicones polyoxyalkylénées répondant aux formules générales (VII) ou (VIII). Plus particulièrement, ces formules répondent à au moins une des, et de préférence toutes les, conditions suivantes :

- c est égal à 2 ou 3.
- $R_1$ désigne le radical méthyle.

- $R_5$ représente un radical méthyle, un radical acyle en $C_{12}$-$C_{22}$, $CO(CH_2)_dCOOM$.
- a varie de 2 à 25 et plus particulièrement de 2 à 15.
- b est égal à 0.
- n varie de 0 à 100.
- p varie de 1 à 20.

Les silicones polyoxyalkylénées peuvent également être choisies parmi les silicones de formule (X) suivante :

$$([Z\ (R_2SiO)q\ R'_2S1Z0][(\ C_nH_{2n}O)_{r]})_s \qquad (X)$$

ladite formule (X) dans laquelle:

- $R_2$ et $R'_2$, identiques ou différents, représentent un radical hydrocarboné monovalent en $C_1$-$C_{30}$,
- n est un nombre entier allant de 2 à 4,
- q est un nombre supérieur ou égal à 4, de préférence compris entre 4 et 200 et encore plus particulièrement entre 4 et 100.
- r est un nombre supérieur ou égal à 4, de préférence compris entre 4 et 200 et encore plus particulièrement entre 5 et 100.

  - s est un nombre supérieur ou égal à 4, de préférence compris entre 4 et 1 000 et encore plus particulièrement entre 5 et 300.
  - Z représente un groupe organique divalent qui est lié à l'atome de silicium adjacent par une liaison carbone-silicium et au bloc polyoxyalkylène ($C_nH_{2n}O$) par un atome d'oxygène,
  - le poids moléculaire moyen de chaque bloc siloxane est compris entre environ 400 et environ 10 000, celui de chaque bloc polyoxyalkylène étant compris entre environ 300 et environ 10 000,
  - les blocs siloxane représentent de 10% environ à 95% environ en poids du copolymère bloc,
  - le poids moléculaire moyen en nombre du copolymère bloc pouvant aller de 2 500 à 1 000 000 et de préférence compris entre 3 000 et 200 000 et encore plus particulièrement entre 6 000 et 100 000.

$R_2$ et $R'_2$ sont préférentiellement choisis parmi le groupe comprenant les radicaux alkyles linéaires ou ramifiés comme par exemple les radicaux méthyle, éthyle, propyle, butyle, pentyle, hexyle, octyle, décyle, dodécyle, les radicaux aryles comme par exemple phényle, naphtyle, les radicaux aralkyls ou alkylaryles comme par exemple benzyle, phényléthyle, les radicaux tolyle, xylyle.

Z est de préférence -R"-, -R"-CO-, -R"-NHCO-, -R"-NH-CO-NH-R"'-, -R"-OCONH-R"'-NHCO-, où R" est un groupe alkylène divalent, linéaire ou ramifié en $C_1$-$C_6$, comme par exemple l'éthylène, le propylène ou le butylène, linéaire ou ramifié et R"' est un groupe alkylène divaient ou un groupe arylène divalent comme $-C_6H_4-$, $-C_6H_4-C_6H_4-$, $-C_6H_4-CH_2-C_6H_4-$, $-C_6H_4-C(CH_3)_2C_6H_4-$.

Encore plus préférentiellement, Z représente un radical alkylène divalent, plus particulièrement le radical $-C_3H_6-$ ou le radical $C_4H_8$, linéaires ou ramifiés.

La préparation des copolymères blocs est décrite dans la demande européenne EP 0 492 657 A1, dont l'enseignement est inclus dans la présente description.

De tels produits sont par exemple commercialisés sous la dénomination SILICONE FLUID FZ-2172 par la société OSI.

Les silicones utilisées peuvent se présenter sous forme de solutions aqueuses, c'est-à-dire solubilisée ou éventuellement sous forme de dispersions ou micro-dispersion, ou d'émulsions aqueuses.

La ou les silicones utilisables dans la composition cosmétique peuvent aussi être des gommes de silicone.

Les gommes de silicone utilisables dans la composition cosmétique sont notamment des polydiorganosiloxanes ayant des poids moléculaires moyens en poids élevés compris entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :

- polydiméthylsiloxane
- les gommes polydiméthylsiloxanes/méthylvinylsiloxane,
- polydiméthylsiloxane/diphénylméthylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :

- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6} m^2/s$. Ce produit comporte de préférence 15 % de gomme SE 30 et 85 % d'une huile SF 96.

La ou les silicones utilisables dans la composition cosmétique peuvent aussi être des silicones aminées.

Par silicone aminé, on entend toute silicone comportant au moins une fonction amine primaire, secondaire, tertiaire ou un groupement ammonium quaternaire.

Les silicones aminées utilisées dans la composition cosmétique selon la présente invention sont choisies parmi :

(a) les composés répondant à la formule (XI) suivante :

$$(R^1)_a(T)_{3-a}\text{-Si}[OSi(T)_2]_n\text{-}[OSi(T)_b(R^1)_{2-b}]_m\text{-}OSi(T)_{3-a}\text{-}(R^1)_a \qquad (XI)$$

dans laquelle,

T est un atome d'hydrogène, ou un radical phényle, hydroxyle (-OH), ou alkyle en $C_1$-$C_8$, et de préférence méthyle ou alcoxy en $C_1$-$C_8$, de préférence méthoxy,
a désigne le nombre 0 ou un nombre entier de 1 à 3, et de préférence 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
$R_1$ est un radical monovalent de formule $-C_qH_{2q}L$ dans laquelle q est un nombre de 2 à 8 et L est un groupement aminé éventuellement quaternisé choisi parmi les groupements :

- $N(R^2)\text{-}CH_2\text{-}CH_2\text{-}N(R^2)_2$ ;
- $N(R^2)_2$ ; $-N^+(R^2)_3\ Q^-$ ;
- $N^+(R^2)\ (H)2\ Q^-$ ;
- $N^+(R^2)_2HQ-$ ,
- $N(R^2)\text{-}CH_2\text{-}CH_2\text{-}N^+(R^2)(H)_2\ Q^-$,

dans lesquels $R^2$ peut désigner un atome d'hydrogène, un phényle, un benzyle, ou un radical hydrocarboné saturé monovalent, par exemple un radical alkyle en $C_1$-$C_{20}$, et Q- représente un ion halogénure tel que par exemple fluorure, chlorure, bromure ou iodure.
En particulier, les silicones aminées correspondant à la définition de la formule (XI) sont choisies parmi les composés correspondant à la formule suivante :

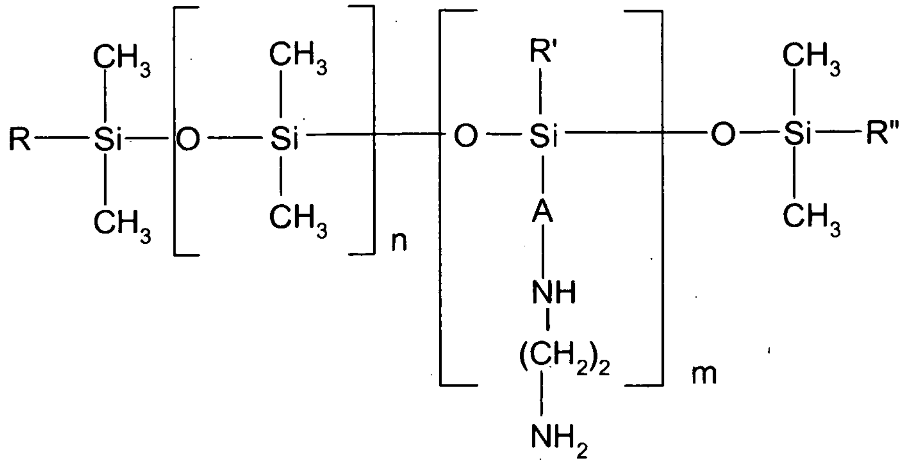

(XII)

dans laquelle R, R', R", identiques ou différents, désignent un radical alkyle en $C_1$-$C_4$, de préférence $CH_3$ ; un radical alcoxy en $C_1$-$C_4$, de préférence méthoxy ; ou OH ; A représente un radical alkylène, linéaire ou ramifié, en $C_3$-$C_8$, de préférence en $C_3$-$C_6$; m et n sont des nombres entiers dépendant du poids moléculaire et dont la somme est comprise entre 1 et 2000.

Selon une première possibilité, R, R', R", identiques ou différents, représentent un radical alkyle en $C_1$-$C_4$ ou hydroxyle, A représente un radical alkylène en $C_3$ et m et n sont tels que la masse moléculaire moyenne en poids du composé est comprise entre 5 000 et 500 000 environ. Les composés de ce type sont dénommés dans le dictionnaire CTFA, "amodiméthicone".

Selon une deuxième possibilité, R, R', R", identiques ou différents, représentent un radical alcoxy en $C_1$-$C_4$ ou hydroxyle, l'un au moins des radicaux R ou R" est un radical alcoxy et A représente un radical alkylène en $C_3$. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et $10^6$. Plus particulièrement, n est compris entre 0 et 999 et m est compris entre 1 et 1000, la somme de n et m étant comprise entre 1 et 1000.

Dans cette catégorie de composés, on peut citer entre autres, le produit Belsil®ADM 652, commercialisée par Wacker.

Selon une troisième possibilité, R, R", différents, représentent un radical alcoxy en $C_1$-$C_4$ ou hydroxyle, l'un au moins des radicaux R, R" est un radical alcoxy, R' représente un radical méthyle et A représente un radical alkylène en $C_3$. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.

Plus particulièrement, on peut citer le produit FluidWR® 1300, commercialisé par Wacker.

Selon une quatrième possibilité, R, R" représentent un radical hydroxyle, R' représente un radical méthyle et A est un radical alkylène, en $C_4$-$C_8$, de préférence en $C_4$. Par ailleurs, m et n sont tels que la masse moléculaire moyenne en poids du composé est comprise entre 2000 et $10^6$. Plus particulièrement, n est compris entre 0 et 1999 et m est compris entre 1 et 2000, la somme de n et m étant comprise entre 1 et 2000.

Un produit de ce type est notamment commercialisé sous la dénomination DC28299 par Dow Corning.

Notons que la masse moléculaire de ces silicones est déterminée par chromatographie par perméation de gel (température ambiante, étalon polystyrène ; colonnes $\mu$ styragem ; éluant THF ; débit de 1 mm/m ; on injecte 200 $\mu$l d'une solution à 0,5 % en poids de silicone dans le THF et l'on effectue la détection par réfractométrie et UV-métrie).

Un produit correspondant à la définition de la formule (XI) est en particulier le polymère dénommé dans le dictionnaire CTFA "triméthylsilylamodiméthicone", répondant à la formule (XIII) suivante:

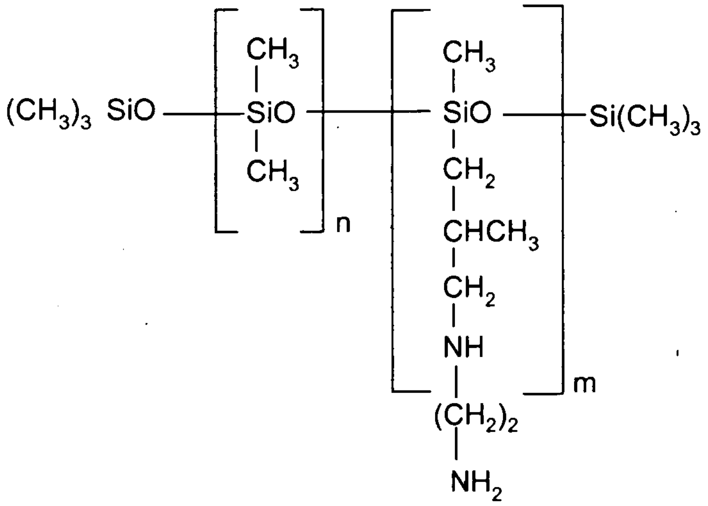

(XIII)

dans laquelle n et m ont les significations données ci-dessus conformément à la formule (XI).

De tels composés sont décrits par exemple dans EP 95238; un composé de formule (XIII) est par exemple vendu sous la dénomination Q2-8220 par la société OSI.

(b) les composés répondant à la formule (XIV) suivante :

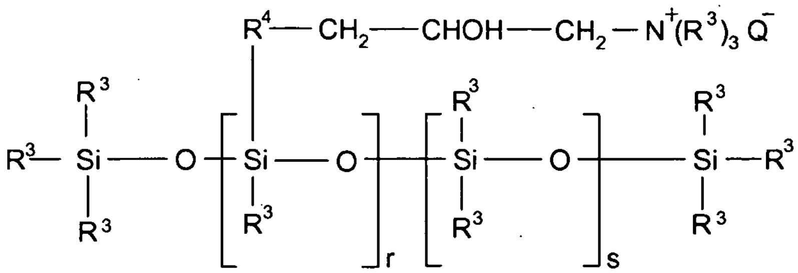

(XIV)

dans laquelle,

$R^3$ représente un radical hydrocarboné monovalent en $C_1$-$C_{18}$, et en particulier un radical alkyle en $C_1$-$C_{18}$, ou alcényle en $C_2$-$C_{18}$, par exemple méthyle ;
$R^4$ représente un radical hydrocarboné divalent, notamment un radical alkylène en $C_1$-$C_{18}$ ou un radical alkylèneoxy divalent en $C_1$-$C_{18}$ , par exemple en$C_1$-$C_8$,
$Q^-$ est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

De tels composés sont décrits plus particulièrement dans le brevet US 4185087.

Un composé entrant dans cette classe est celui vendu par la Société Union Carbide sous la dénomination "Ucar Silicone ALE 56".

c) les silicones ammonium quaternaire de formule (XV) :

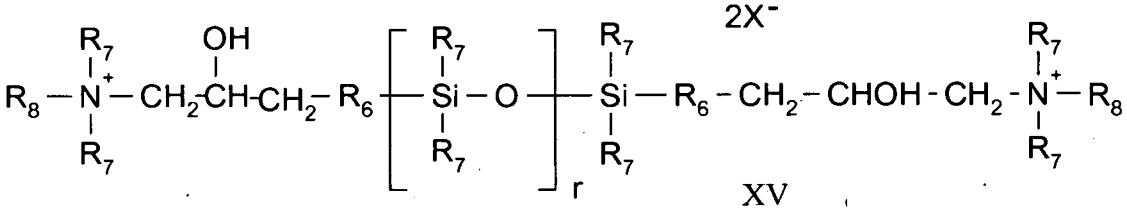

XV

dans laquelle :

$R_7$, identiques ou différents, représentent un radical hydrocarboné monovalent ayant de 1 à 18 atomes de carbone, et en particulier un radical alkyle en $C_1$-$C_{18}$, un radical alcényle en $C_2$-$C_{18}$ ou un cycle comprenant 5 ou 6 atomes de carbone, par exemple méthyle ;
$R_6$ représente un radical hydrocarboné divalent, notamment un radical alkylène en $C_1$-$C_{18}$ ou un radical alkylèneoxy divalent en $C_1$-$C_{18}$, par exemple en $C_1$-$C_8$ relié au Si par une liaison SiC;
$R_8$, identiques ou différents, représentent un atome d'hydrogène, un radical hydrocarboné monovalent ayant de 1 à 18 atomes de carbone, et en particulier un radical alkyle en $C_1$-$C_{18}$, un radical alcényle en $C_2$-$C_{18}$ , un radical -$R_6$-NHCOR$_7$ ,
X- est un anion tel qu'un ion halogénure, notamment chlorure ou un anion d'acide organique (acétate ...);
r représente une valeur statistique moyenne de 2 à 200 et en particulier de 5 à 100 ;

Ces silicones sont par exemple décrites dans la demande EP-A-0530974.
d) les silicones aminées de formule (XVI) :

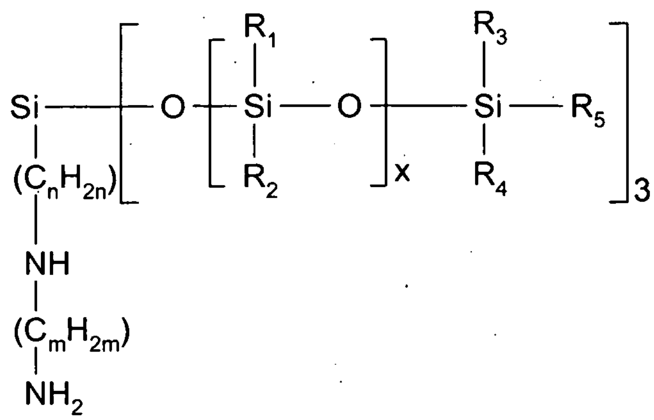

dans laquelle :

- $R_1$, $R_2$, $R_3$ et $R_4$, identiques ou différents, désignent un radical alkyle en $C_1$-$C_4$ ou un groupement phényle,
- $R_5$ désigne un radical alkyle en $C_1$-$C_4$ ou un groupement hydroxyle,
- n est un entier variant de 1 à 5,
- m est un entier variant de 1 à 5,

et dans laquelle x est choisi de manière telle que l'indice d'amine soit compris entre 0,01 et 1 meq/g.

Les silicones particulièrement préférées sont les polysiloxanes à groupements aminés tels que les amodiméthicones ou les triméthylsilylamodiméthicones (CTFA 4ème édition 1997), et encore plus particulièrement les silicones à groupements ammonium quaternaire.

Lorsque ces composés sont mis en oeuvre, une forme de réalisation particulièrement intéressante est leur utilisation conjointe avec des agents de surface cationiques et/ou non ioniques.

A titre exemple, on peut utiliser le produit vendu sous la dénomination "Emulsion Cationique DC 929" par la Société Dow Corning, qui comprend, outre l'amodiméthicone, un agent de surface cationique comprenant un mélange de produits répondant à la formule :

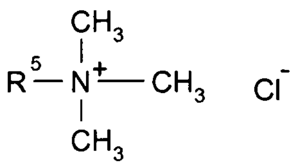

dans laquelle, $R^5$ désigne des radicaux alcényle et/ou alcoyle en $C_{14}$-$C_{22}$ dérivés des acides gras du suif, et connu sous la dénomination CTFA "tallowtrimonium chloride",en association avec un agent de surface non ionique de formule :

$C_9H_{19}$-$C_6H_4$-$(OC_2H_4)_{10}$-OH, connu sous la dénomination CTFA "Nonoxynol 10".

On peut également utiliser par exemple le produit vendu sous la dénomination "Emulsion Cationique DC 939" par la Société Dow Corning, qui comprend, outre l'amodiméthicone, un agent de surface cationique qui est le chlorure de triméthylcétylammonium et un agent de surface non ionique de formule : $C_{13}H_{27}$-$(OC_2H_4)_{12}$-OH, connu sous la dénomination CTFA "tridéceth-12".

Un autre produit commercial utilisable selon l'invention est le produit vendu sous la dénomination "Dow Corning Q2 7224" par la Société Dow Corning, comportant en association le triméthylsilylamodiméthicone de formule (C) décrite ci-dessus, un agent de surface non ionique de formule : $C_8H_{17}$-$C_6H_4$-$(OCH_2CH_2)_{40}$-OH, connu sous la dénomination CTFA "octoxynol-40", un second agent de surface non ionique de formule: $C_{12}H_{25}$-$(OCH_2$-$CH_2)_6$-OH, connu sous la dénomination CTFA "isolaureth-6", et du propylèneglycol.

Le ou les silicones sont présents dans la composition dans une quantité variant de 0,1 à 30 %, de préférence en une quantité variant de 0,2 à 20 %, et encore plus préférentiellement dans une quantité variant de 0,5 à 10 % en poids, par rapport au poids total de la composition.

La composition cosmétique selon l'invention peut comprendre en outre un corps gras non siliconé tels que les huiles végétales, animales, minérales et synthétiques, les alcools gras, les acides gras et les cires.

Le ou les corps gras sont présents dans la composition dans une quantité variant de 0,1 à 30 %, de préférence en une quantité variant de 0,2 à 20 %, et encore plus préférentiellement dans une quantité variant de 0,5 à 10 % en poids, par rapport au poids total de la composition.

Par alcool gras, on entend au sens de la présente invention, tout alcool gras pur saturé ou insaturé, linéaire ou ramifié comportant au moins 8 atomes de carbone. L'alcool gras peut être oxyalkyléné ou glycérolé.

L'alcool gras peut présenter la structure R-OH, dans laquelle R désigne un radical saturé ou insaturé, linéaire ou ramifié, comportant de 8 à 40 atomes de carbone et de préférence de 8 à 30 ; R désigne de préférence un groupement alkyle en $C_{12}$-$C_{24}$ ou alkényle en $C_{12}$-$C_{24}$. R peut être substitué par un ou plusieurs groupements hydroxy.

A titre d'exemple d'alcools gras, on peut citer les alcools laurique, cétylique, dodécylique, décylique, stéarylique, oléïque, béhénique, linoléique, undécylénique, palmitoléïque, arachidonique, érucique et leurs mélanges.

L'alcool gras peut représenter un mélange d'alcools gras, ce qui signifie que dans un produit commercial peuvent coexister plusieurs espèces d'alcools gras, sous forme d'un mélange.

A titre de mélange d'alcools gras, on peut citer l'alcool cétylstéarylique ou cétéarylique.

Avantageusement, l'alcool gras non oxyalkyléné est solide ou pâteux à la température de 25°C. Par « alcool gras solide ou pâteux à 25°C », on entend au sens de la présente invention un alcool gras présentant une viscosité mesurée avec un rhéomètre avec un taux de cisaillement de $1s^{-1}$ supérieure ou égale à 1 Pa.s.

De préférence, les alcools gras utilisés dans la composition cosmétique selon l'invention sont l'alcool cétylique et l'alcool cétéarylique.

Par acides gras, on entend au sens de la présente invention, tout acide carboxylique pur saturé ou insaturé, linéaire ou ramifié comportant au moins 8 atomes de carbone. A titre d'exemples d'acide gras, on peut citer l'acide laurique, l'acide oléique.

La composition cosmétique peut comprendre en outre un ou plusieurs polymères fixants additionnels différents du matériau fixant de la présente invention.

Par polymère fixant, on entend au sens de la présente invention tout polymère permettant de conférer une forme ou de maintenir une forme ou une coiffure donnée.

Les polymères fixants utilisables dans la composition cosmétique selon l'invention sont notamment choisis parmi les polymères cationiques, anioniques, amphotères, non ioniques et leurs mélanges.

Par « polymère cationique », on entend au sens de la présente invention, tout polymère comprenant des groupements cationiques et/ou des groupements ionisables en groupements cationiques.

Les polymères fixants cationiques utilisables dans la composition cosmétique selon l'invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre

comprise entre 500 et environ 5 000 000, et de préférence entre 1 000 et 3 000 000.

Parmi ces polymères, on peut citer plus particulièrement les polymères cationiques suivants :

(1) les homopolymères ou copolymères d'esters ou d'amides acryliques ou méthacryliques, à fonctions aminées, comportant au moins un des motifs de formules suivantes :

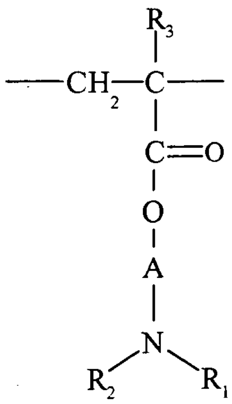
(A)
,
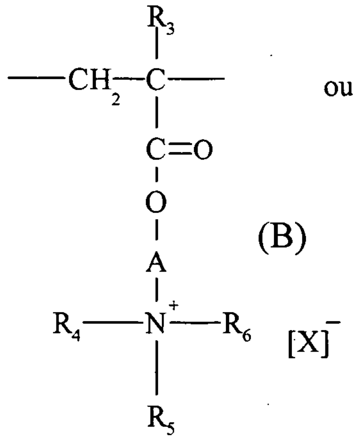
(B)
ou
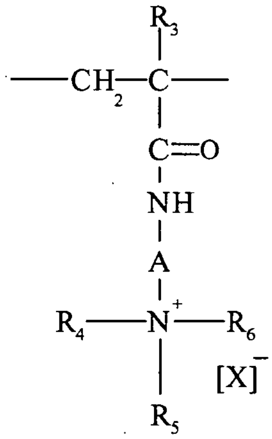
(C)

dans lesquelles:

$R_1$ et $R_2$, identiques ou différents, représentent chacun un atome d'hydrogène ou un groupe alkyle ayant de 1 à 6 atomes de carbone ;
$R_3$ désigne un atome d'hydrogène ou un groupe $CH_3$ ;
A est un groupe alkyle linéaire ou ramifié, comportant de 1 à 6 atomes de carbone ou un groupe hydroxyalkyle comportant de 1 à 4 atomes de carbone ;
$R_4$, $R_5$, $R_6$, identiques ou différents, représentent un groupe alkyle ayant de 1 à 18 atomes de carbone ou un groupe benzyle ;
X désigne un anion méthosulfate ou un halogénure tel que chlorure ou bromure.

Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur ($C_{1-4}$), des groupes dérivés des acides acryliques ou méthacryliques ou de leurs esters, de vinyllactames tels que la vinylpyrrolidone ou le vinylcaprolactame, d'esters vinyliques.
Ainsi, parmi ces copolymères de la famille (1), on peut citer :

- les copolymères d'acrylamide et de méthacrylate de diméthylaminoéthyle quaternisés au sulfate de diméthyle ou avec un halogénure de diméthyle, tels que celui vendu sous la dénomination HERCOFLOC® par la société HERCULES,
- les copolymères d'acrylamide et de chlorure de méthacryloyloxyéthyltriméthylammonium décrits, par exemple, dans la demande de brevet EP-A-080976 et vendus sous la dénomination BINA QUAT P 100 par la société CIBA GEIGY,
- les copolymères d'acrylamide et de méthosulfate de méthacryloyloxyéthyltriméthylammonium, tels que celui vendu sous la dénomination RETEN par la société HERCULES,
- les copolymères vinylpyrrolidone/acrylate ou méthacrylate de dialkylaminoalkyle quaternisés ou non, tels que les produits vendus sous la dénomination "GAFQUAT®" par la société ISP comme, par exemple, "GAFQUAT® 734" ou "GAFQUAT® 755", ou bien les produits dénommés "COPOLYMER® 845, 958 et 937". Ces polymères sont décrits en détail dans les brevets français Nos 2 077 143 et 2 393 573,
- les terpolymères méthacrylate de diméthylaminoéthyle/ vinylcaprolactame/vinylpyrrolidone tels que le produit commercialisé sous la dénomination GAFFIX® VC 713 par la société ISP, et
- les copolymères vinylpyrrolidone/méthacrylamide de diméthylaminopropyle quaternisé tels que notamment le produit commercialisé sous la dénomination "GAFQUAT® HS 100" par la société ISP ;

(2) les polysaccharides cationiques, de préférence à ammonium quaternaire, tels que ceux décrits dans les brevets

américains 3 589 578 et 4 031 307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL ;

(3) les copolymères quaternaires de vinylpyrrolidone et de vinylimidazole ;

(4) les chitosanes ou leurs sels ; les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90,5 % en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL.

(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyloxyéthyl-triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule :

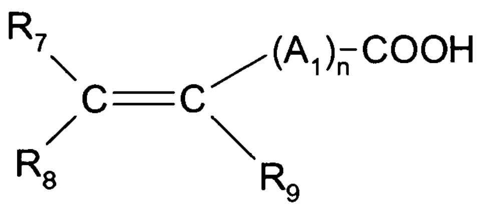

(I)

dans laquelle n est un nombre entier de 0 à 10, $A_1$ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, $R_7$ désigne un atome d'hydrogène, un groupement phényle ou benzyle, $R_8$ désigne un atome d'hydrogène, un groupement alkyle inférieur ou carboxyle, $R_9$ désigne un atome d'hydrogène, un groupement alkyle inférieur, un groupement $-CH_2-COOH$, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :

A) Les homo- ou copolymères d'acide acrylique ou méthacrylique ou leurs sels et en particulier les produits vendus sous les dénominations VERSICOL® E ou K par la société ALLIED COLLOID, et ULTRAHOLD® par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques.

B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois nos 75370 et 75371 ou proposés sous la dénomination QUADRAMER par la Société AMERICAN CYANAMID. On peut également citer les terpolymères acide méthacrylique/acrylate d'éthyle/acrylate de tertiobutyle tels que le produit commercialisé sous la dénomination LUVIMER® 100 P par la société BASF.

On peut aussi citer les copolymères acide méthacrylique/acide acrylique/acrylate d'éthyle/méthacrylate de méthyle en dispersion aqueuse, commercialisé sous la dénomination AMERHOLD® DR 25 par la société AMERCHOL.

C) Les copolymères d'acide crotonique, tels que ceux comportant dans leur chaîne des motifs acétate ou propionate

de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n$^{os}$ 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch.

D) Les copolymères d'acides ou d'anhydrides carboxyliques monoinsaturés en $C_4$-$C_8$ choisis parmi :

- les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US nos 2 047 398, 2 723 248, 2 102 113, le brevet GB n° 839 805. Des produits commerciaux sont notamment ceux vendus sous les dénominations GANTREZ® AN ou ES par la société ISP.
- les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
  les fonctions anhydrides de ces copolymères étant éventuellement monoestérifiées ou monoamidifiées.

Ces polymères sont par exemple décrits dans les brevets français nos 2 350 384 et 2 357 241 de la demanderesse.

E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :

- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous les dénominations Flexan® 500 et Flexan® 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par Henkel.

De préférence, les polymères fixants anioniques sont choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique vendus notamment sous la dénomination LUVISET CA 66 par la société BASF et les copolymères acétate de vinyle/acide crotonique greffés par du polyéthylèneglycol vendus sous la dénomination ARISTOFLEX® A par la société BASF.

Parmi les polymères fixants anioniques cités ci-dessus, on préfère plus particulièrement utiliser dans le cadre de la présente invention les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH,

les copolymères d'acide méthacrylique et d'acrylate d'éthyle vendus sous la dénomination LUVIMER® MAEX OU MAE par la société BASF.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-$\alpha,\beta$-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :

(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet américain n° 3 836 537.

(2) les polymères comportant des motifs dérivant :

a) d'au moins un monomère choisi parmi les acrylamides ou les méthacrylamides substitués sur l'atome d'azote par un groupe alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
Les comonomères basiques préférés sont des méthacrylates d'aminoéthyle, de butylaminoéthyle, de N,N'-diméthylaminoéthyle, de N-tertio-butylaminoéthyle.
On utilise particulièrement les copolymères dont la dénomination CTFA (4ème Ed., 1991) est octylacrylamide/ acrylates/ butylaminoethyl methacrylate copolymer, tels que les produits vendus sous la dénomination AMPHOMER® ou LOVOCRYL® 47 par la société NATIONAL STARCH.

(3) les polyaminoamides réticulés et acylés partiellement ou totalement dérivant de poyaminoamides de formule générale :

$$-\!\!\left[CO-R_{10}-CO-Z\right]\!\!- \qquad (XVII)$$

dans laquelle $R_{10}$ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :

a) dans les proportions de 60 à 100 % en moles, le groupe

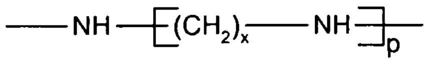

(XVIII)

où x=2 et p=2 ou 3, ou bien x=3 et p=2
ce groupe dérivant de la diéthylène-triamine, de la triéthylène-tétraamine ou de la dipropylène-triamine;
b) dans les proportions de 0 à 40 % en moles, le groupe (XVIII) ci-dessus, dans lequel x=2 et p=1 et qui dérive de l'éthylène-diamine, ou le groupe dérivant de la pipérazine :

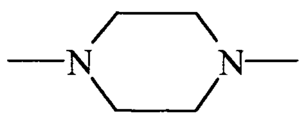

c) dans les proportions de 0 à 20 % en moles, le groupe $-NH-(CH_2)_6-NH-$ dérivant de l'hexaméthylènediamine,

ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.
Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule :

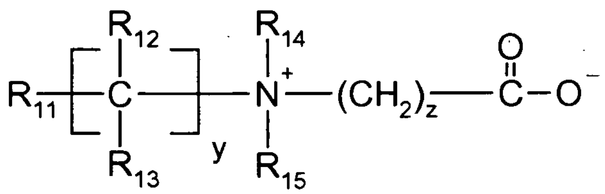

dans laquelle $R_{11}$ désigne un groupement insaturé polymérisable tel qu'un groupement acrylate, méthacrylate, acrylamide ou méthacrylamide, y et z représentent un nombre entier de 1 à 3, $R_{12}$ et $R_{13}$ représentent un atome d'hydrogène, un groupe méthyle, éthyle ou propyle, $R_{14}$ et $R_{15}$ représentent un atome d'hydrogène ou un groupe alkyle de telle façon que la somme des atomes de carbone dans $R_{14}$ et $R_{15}$ ne dépasse pas 10.
Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
A titre d'exemple, on peut citer les copolymères méthacrylate de méthyle/diméthyl-carboxyméthylammonioéthylméthacrylate de méthyle, tels que le produit vendu sous la dénomination DIAFORMER Z301 par la société SANDOZ.
(5) les polymères dérivés du chitosane comportant des motifs monomères répondant aux formules suivantes :

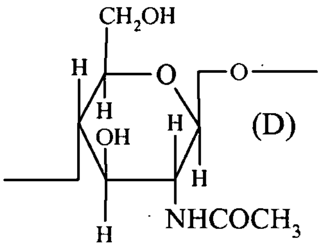
(D)

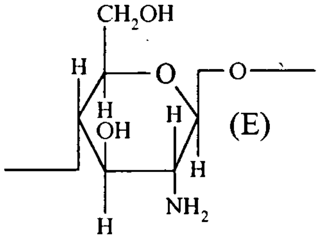
(E)

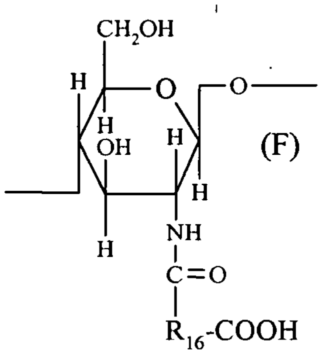
(F)

le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), $R_{16}$ représente un groupe de formule :

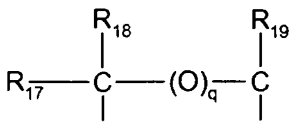

dans laquelle si q=0, $R_{17}$, $R_{18}$ et $R_{19}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes $R_{17}$, $R_{18}$ et $R_{19}$ étant dans ce cas un atome d'hydrogène ;
ou si q=1, $R_{17}$, $R_{18}$ et $R_{19}$ représentent chacun un atome d'hydrogène, ainsi que les sels formés par ces composés avec des bases ou des acides.
(6) Les polymères comportant des motifs répondant à la formule générale (XIX) qui sont, par exemple, décrits dans le brevet français 1 400 366 :

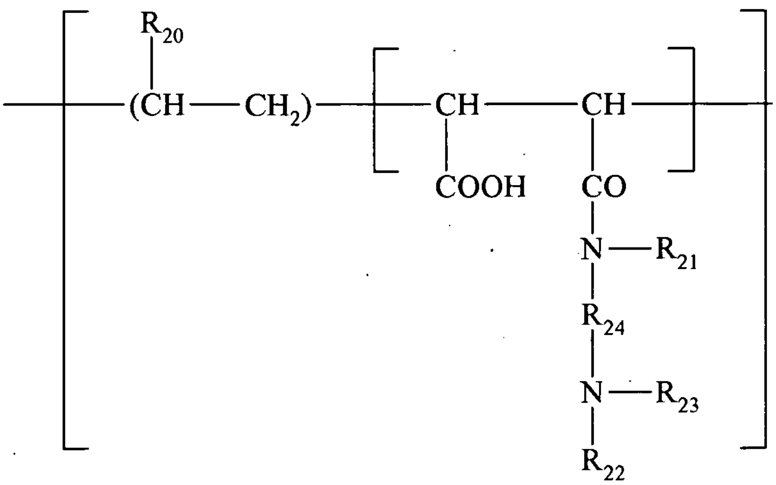
(XIX)

dans laquelle $R_{20}$ représente un atome d'hydrogène, un groupe $CH_3O$, $CH_3CH_2O$, phényle, $R_{21}$ désigne un atome d'hydrogène ou un groupe alkyle inférieur tel que méthyle, éthyle, $R_{22}$ désigne un atome d'hydrogène ou un groupe alkyle inférieur en $C_1$-$C_6$ tel que méthyle, éthyle, $R_{23}$ désigne un groupe alkyle inférieur en $C_1$-$C_6$ tel que méthyle, éthyle ou un groupe répondant à la formule : $-R_{24}-N(R_{22})_2$, $R_{24}$ représentant un groupement $-CH_2-CH_2-$,

$-CH_2-CH_2-CH_2-$, $-CH_2-CH(CH_3)-$, $R_{22}$ ayant les significations mentionnées ci-dessus.

(7) Les polymères dérivés de la N-carboxyalkylation du chitosane comme le N-carboxyméthyl-chitosane ou le N-carboxybutyl-chitosane, vendu sous la dénomination "EVALSAN" par la société JAN DEKKER.

(8) Les polymères amphotères du type -D-X-D-X choisis parmi:

a) les polymères obtenus par action de l'acide chloracétique ou le chloracétate de sodium sur les composés comportant au moins un motif de formule :

-D-X-D-X-D- (XX)

où D désigne un groupe

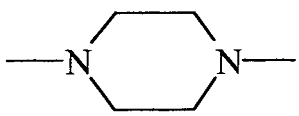

et X désigne le symbole E ou E', E ou E' identiques ou différents désignant un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.

b) Les polymères de formule :

-D-X-D-X- (XXI')

où D désigne un groupe

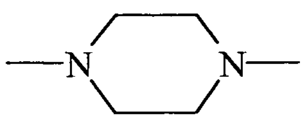

et X désigne le symbole E ou E' et au moins une fois E' ; E ayant la signification indiquée ci-dessus et E' étant un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) les copolymères alkyl($C_1$-$C_5$)vinyléther/anhydride maléique modifiés partiellement par semiamidification avec une N,N-dialkylaminoalkylamine telle que la N,N-diméthylaminopropylamine ou par semiestérification avec un N, N-dialkylaminoalcanol. Ces copolymères peuvent également comporter d'autres comonomères vinyliques tels que le vinylcaprolactame.

Parmi les polymères fixants amphotères décrits ci-dessus, les plus particulièrement préférés selon l'invention sont ceux de la famille (3) tels que les copolymères dont la dénomination CTFA est Octylacrylamide/ acrylates/butylaminoethylmethacrylate copolymer, tels que les produits vendus sous les dénominations AMPHOMER®, AMPHOMER® LV 71 ou LOVOCRYL® 47 par la société NATIONAL STARCH et ceux de la famille (4) tels que les copolymères méthacrylate de méthyle/diméthylcarboxyméthylammonioéthylméthacrylate de méthyle vendu par exemple sous la dénomination DIAFORMER® Z301 par la société SANDOZ.

Les polymères fixants non ioniques utilisables selon la présente invention sont choisis, par exemple, parmi :

- les polyalkyloxazolines ;

- les homopolymères d'acétate de vinyle;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth) acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHODIA CHIMIE ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol® PLUS par la société BASF ; et
- les copolymères de vinyllactame tels qu'un copolymère poly(vinylpyrrolidone/vinyllactame) vendu sous le nom commercial Luvitec® VPC 55K65W par la société BASF, les copolymères poly(vinylpyrrolidone/acétate de vinyle) comme ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol® VAP 343 par la société BASF.

Les groupes alkyle des polymères non ioniques mentionnés ci-dessus ont, de préférence, de 1 à 6 atomes de carbone.

On peut également utiliser comme polymères fixants, des polyuréthanes fonctionnalisés ou non, siliconés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous les dénominations LUVISET PUR® et LUVISET® Si PUR par la société BASF.

Le ou les polymères fixants additionnels est ou sont présents dans la composition cosmétique selon l'invention en une quantité variant de 0,01 à 20 % en poids, de préférence de 0,05 à 15 % en poids, et encore plus préférentiellement de 0,1 à 10 % en poids, par rapport au poids total de la composition cosmétique.

La composition selon l'invention peut également contenir des tensio-actifs.

Le ou les tensioactifs ioniques utilisés dans la composition cosmétique peuvent être des tensioactifs cationiques.

A titre d'exemple de tensioactifs cationiques utilisables dans la composition cosmétique, on peut notamment citer les sels d'amines grasses primaires, secondaires ou tertiaires, éventuellement polyoxyalkylénées, les sels d'ammonium quaternaire, et leurs mélanges.

A titre de sels d'ammonium quaternaires, on peut notamment citer, par exemple :

- ceux qui présentent la formule générale (I) suivante :

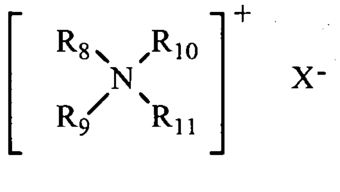

(I)

dans laquelle les radicaux R8 à R11, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes.

Les radicaux aliphatiques sont par exemple choisis parmi les radicaux alkyle, alcoxy, polyoxyalkylène (C2-C6), alkylamide, alkyl(C 12-C22)amidoalkyle(C2-C6), alkyl(C 12-C22)acétate, hydroxyalkyle, comportant environ de 1 à 30 atomes de carbone ; X est un anion choisi dans le groupe des halogénures, phosphates, acétates, lactates, alkyl(C2-C6)sulfates, alkyl- ou alkylaryl-sulfonates ;

- les sels d'ammonium quaternaire de l'imidazoline, comme par exemple ceux de formule (II) suivante :

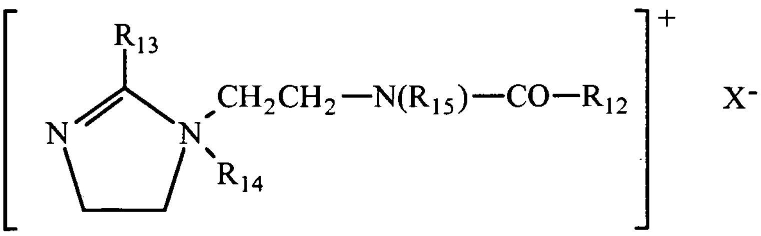

(II)

dans laquelle R12 représente un radical alcényle ou alkyle comportant de 8 à 30 atomes de carbone, par exemple dérivés des acides gras du suif, R13 représente un atome d'hydrogène, un radical alkyle en C1-C4 ou un radical alcényle ou alkyle comportant de 8 à 30 atomes de carbone, R14 représente un radical alkyle en C1-C4, R15 représente un atome d'hydrogène, un radical alkyle en C1-C4, X- est un anion choisi dans le groupe des halogénures, phosphates, acétates, lactates, alkylsulfates, alkyl- ou alkylaryl-sulfonates. De préférence, R12 et R13 désignent un mélange de radicaux alcényle ou alkyle comportant de 12 à 21 atomes de carbone, par exemple dérivés des acides gras du suif, R14 désigne un radical méthyle, R15 désigne un atome d'hydrogène. Un tel produit est par exemple commercialisé sous la dénomination REWOQUAT® W 75 par la société REWO ;

- les sels de diammonium quaternaire de formule (III) :

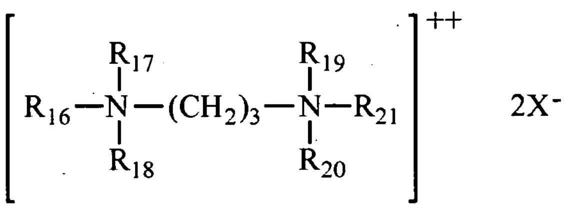

(III)

dans laquelle R16 désigne un radical aliphatique comportant environ de 16 à 30 atomes de carbone, R17, R18, R19, R20 et R21 , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propane suif diammonium ;

- les sels d'ammonium quaternaire contenant au moins une fonction ester, tels que ceux de formule (IV) suivante :

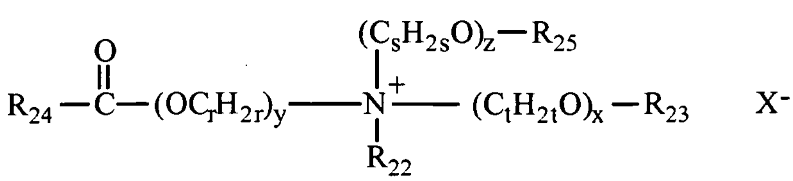

(IV)

dans laquelle :

R22 est choisi parmi les radicaux alkyles en C1-C6 et les radicaux hydroxyalkyles ou dihydroxyalkyles en C1-C6 ;

R23 est choisi parmi :

- le radical

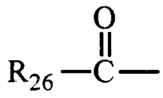

- les radicaux R27 hydrocarbonés en C1-C22, linéaires ou ramifiés, saturés ou insaturés,
- l'atome d'hydrogène,

R25 est choisi parmi :

- le radical

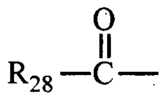

- les radicaux R29 hydrocarbonés en C1-C6, linéaires ou ramifiés, saturés ou insaturés,
- l'atome d'hydrogène,

R24, R26 et R28, identiques ou différents, sont choisis parmi les radicaux hydrocarbonés en C7-C21, linéaires ou ramifiés, saturés ou insaturés ;
r, s et t, identiques ou différents, sont des entiers valant de 2 à 6;
y est un entier valant de 1 à 10 ;
x et z, identiques ou différents, sont des entiers valant de 0 à 10 ;
X- est un anion simple ou complexe, organique ou inorganique ;
sous réserve que la somme x + y + z vaut de 1 à 15, que lorsque x vaut 0 alors R23 désigne R27 et que lorsque z vaut 0 alors R25 désigne R29.

Les radicaux alkyles R22 peuvent être linéaires ou ramifiés et plus particulièrement linéaires.
De préférence R22 désigne un radical méthyle, éthyle, hydroxyéthyle ou dihydroxypropyle, et plus particulièrement un radical méthyle ou éthyle.
Avantageusement, la somme x + y + z vaut de 1 à 10.
Lorsque R23 est un radical R27 hydrocarboné, il peut être long et avoir de 12 à 22 atomes de carbone, ou court et avoir de 1 à 3 atomes de carbone.
Lorsque R25 est un radical R29 hydrocarboné, il a de préférence 1 à 3 atomes de carbone.
Avantageusement, R24, R26 et R28, identiques ou différents, sont choisis parmi les radicaux hydrocarbonés en C11-C21, linéaires ou ramifiés, saturés ou insaturés, et plus particulièrement parmi les radicaux alkyle et alcényle en C11-C21, linéaires ou ramifiés, saturés ou insaturés.
De préférence, x et z, identiques ou différents, valent 0 ou 1.
Avantageusement, y est égal à 1.
De préférence, r, s et t, identiques ou différents, valent 2 ou 3, et encore plus particulièrement sont égaux à 2.
L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.
L'anion X- est encore plus particulièrement le chlorure ou le méthylsulfate.
On utilise plus particulièrement dans la composition selon l'invention, les sels d'ammonium de formule (IV) dans laquelle :

- R22 désigne un radical méthyle ou éthyle,
- x et y sont égaux à 1 ;
- z est égal à 0 ou 1 ;

- r, s et t sont égaux à 2 ;
- R23 est choisi parmi :

  - le radical

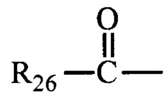

  - les radicaux méthyle, éthyle ou hydrocarbonés en C14-C22,
  - l'atome d'hydrogène ;
  - R25 est choisi parmi :

  - le radical

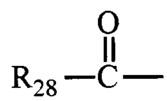

  - l'atome d'hydrogène ;
  - R24, R26 et R28, identiques ou différents, sont choisis parmi les radicaux hydrocarbonés en C13-C17, linéaires ou ramifiés, saturés ou insaturés, et de préférence parmi les radicaux alkyles et alcényles en C13-C17, linéaires ou ramifiés, saturés ou insaturés.

Avantageusement, les radicaux hydrocarbonés sont linéaires.

On peut citer par exemple les composés de formule (IV) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxy éthyl-méthylammonium, de monoacyloxyéthyl-hydroxyéthyl-diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthane sulfonate de méthyle, le paratoluènesulfonate de méthyle, la chlorhydrine du glycol ou du glycérol.

De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthyl sulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (I), on préfère d'une part, les chlorures de tétraalkylammonium comme, par exemple, les chlorures de dialkyldiméthylammonium ou d'alkyltrimé thylammonium dans lesquels le radical alkyle comporte environ de 12 à 22 atomes de carbone, en particulier les chlorures de béhényltriméthyl ammonium, de distéaryldiméthylammonium, de cétyltriméthylammo nium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL® 70 par la société VAN DYK.

Les tensioactifs cationiques particulièrement préférés dans la composition de l'invention sont choisis parmi les sels d'ammonium quaternaire, et en particulier parmi le chlorure de cétyltriméthyl ammonium, le chlorure de béhényltriméthylammonium et le chlorure de palmitylamidopropyltriméthylammonium.

Les tensioactifs ioniques utilisables dans la composition cosmétique peuvent être également des tensioactifs anioniques.

Comme tensioactifs anioniques utilisables dans la composition cosmétique selon l'invention, on peut notamment mentionner les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acyl sarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C6-24 et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactosideuroniques et leurs sels ainsi que les acides alkyl (C6-24)éthercarboxyliques polyoxyalkylénés, les acides alkyl(C6-24)aryl(C6-24) éthercarboxyliques polyoxyalkylénés, les acides alkyl(C6-24) amidoéthercarboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et les alkyléthercarboxylates, et leurs mélanges, en particulier sous forme de sels de métaux alcalins ou alcalino-terreux, d'ammonium, d'amine ou d'aminoalcool.

De préférence, le tensioactif ionique est un tensioactif cationique.

Les tensioactifs non ioniques utilisables dans la composition cosmétique de la présente invention sont des composés bien connus en soi (voir notamment à cet égard "Handbook of Surfactants" par M.R. PORTER, éditions Blackie & Son (Glasgow and London), 1991, pp 116-178). Ils sont choisis notamment parmi les alcools, les alphadiols, les alkyl(C1-20)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitane éthoxylés ayant de 2 à 30 motifs d'oxyde d'éthylène, les esters d'acides gras du saccharose, les esters d'acides gras du polyéthylèneglycol, les (alkyle en $C_{6-24}$)polyglycosides, les dérivés de N-(alkyle en $C_{6-24}$)glucamine, les oxydes d'amines tels que les oxydes d'(alkyle en $C_{10-14}$)amines ou les oxydes de N-(acyle en $C_{10-14}$)-aminopropylmorpholine.

Parmi les tensioactifs non ioniques cités ci-dessus, on utilise de préférence les alcools polyéthoxylés, polypropoxylés ou polyglycérolés.

Le ou les tensioactifs sont présents à une concentration allant de 0,01 à 20 % en poids, de préférence à une concentration allant de 0,05 à 10 % en poids, et encore plus préférentiellement à une concentration allant de 0,1 à 5 % en poids, par rapport au poids total de la composition.

La composition cosmétique selon l'invention peut comprendre en outre un ou plusieurs adjuvants cosmétiques choisis parmi les agents conditionneurs de type esters, les agents anti-mousse, les agents hydratants, les agents émollients, les glycols, les plastifiants, les agents épaississants minéraux, les agents épaississants organiques, polymériques ou non, associatifs ou non, les filtres solaires hydrosolubles ou liposolubles, siliconés ou non siliconés, les colorants permanents ou temporaires, les parfums, les peptisants, les conservateurs, les céramides, et pseudo-céramides, les vitamines et les provitamines dont le panthénol, les protéines, les agents séquestrants, les agents solubilisants, les agents alcanisants, les agents acidifiants, les agents anti-corrosion, les agents réducteurs ou anti-oxydants, les agents oxydants, les charges minérales, les paillettes.

L'homme du métier veillera à choisir les éventuels adjuvants et leur quantité de manière à ce qu'ils ne nuisent pas aux propriétés des compositions de la présente invention.

De préférence, le ou les adjuvants cosmétiques sont présents à une concentration allant de 0,001 à 50 % en poids par rapport au poids total de la composition.

Par milieu cosmétiquement acceptable, on entend un milieu compatible avec les matières kératiniques et en particulier les cheveux.

Le milieu cosmétiquement acceptable peut être un milieu alcoolique, aqueux ou hydroalcoolique. Ainsi le milieu peut notamment être constitué uniquement par de l'eau ou d'un monoalcool ou par un mélange d'eau et d'un ou de plusieurs monoalcools cosmétiquement acceptables tels que les alcools inférieurs en $C_1$-$C_4$, les éthers de polyols

présentant un hydroxyle libre et leurs mélanges. De préférence, l'alcool est l'éthanol.

La présente invention concerne aussi un procédé de traitement cosmétique des cheveux, par exemple de coiffage, qui consiste à appliquer une quantité efficace d'une composition décrite ci-dessus, sur les cheveux secs ou humides, à rincer ou non après un éventuel temps de pose ou après un éventuel séchage.

De préférence, la composition est non rincée.

La présente invention concerne également l'utilisation d'une composition cosmétique pour la fixation des fibres.

Les exemples suivants sont donnés à titre illustratif et non limitatif de la présente invention.

EXEMPLES

Les compositions suivantes ont été préparées :

Mousses coiffantes

| | 1* | 2 |
|---|---|---|
| Carraghénane Lambda (WELGEENAN ED 1039-EUROGUM) | 1% | 1,2% |
| APG (PLANTACARE 2000 UP -COGNIS) | 5% | - |
| Tensio-actif ionique ou non ionique (Polysorbate-20) | - | 0,8% |
| Polymère fixant non-ionique (PVP) | - | 3% |
| Ethanol | - | 5% |
| Conservateurs, neutralisant, parfum | QS | QS |
| Eau | QS 100 | QS 100 |
| Gaz propulseur : C02 | 3,5% | 3,5% |
| * Non conforme à l'invention | | |

Cette composition incorporée dans un dispositif aérosol conduit à la formation d'un mousse crémeuse, glissante et peu expansée.

**Revendications**

1. Composition cosmétique pour le traitement des fibres kératiniques, en particulier des fibres kératiniques humaines telles que les cheveux, sous forme aérosol **caractérisée par le fait qu'**elle comprend, dans un milieu cosmétiquement acceptable,

   - au moins un polysaccharide de type carraghénane lambda,
   - du dioxyde de carbone en tant qu'agent propulseur, et
   - au moins un additif choisi parmi une silicone, un corps gras et un polymère fixant différent du polysaccharide de type carraghénane lambda.

2. Composition cosmétique selon la revendication 1, **caractérisée par le fait que** le poids moléculaire (PM) du polysaccharide de type carraghénane lambda est compris entre 100 000 et 1 000 000, de préférence entre 250 000 et 800 000.

3. Composition cosmétique selon la revendication 1 ou 2, **caractérisée par le fait que** le polysaccharide de type carraghénane lambda est présent dans la composition cosmétique en une quantité variant de 0,1 à 30 % en poids, de préférence en une quantité variant de 0,2 à 20 % en poids et encore plus préférentiellement en une quantité variant de 0,5 à 15 % en poids, par rapport au poids total de la composition.

4. Composition selon l'une quelconque des revendications précédentes, **caractérisée par le fait que** l'agent propulseur représente 2 à 70%, de préférence 3 à 50% en poids par rapport au poids total de l'ensemble des compositions contenues dans le dispositif aérosol.

5. Composition selon l'une quelconque des revendications précédentes, **caractérisée par le fait que** le dispositif

aérosol est approprié pour obtenir un débit en matière sèche supérieur ou égal à 20 mg/s, de préférence compris entre 20 et 60 mg/s.

**6.** Composition selon l'une quelconque des revendications précédentes, **caractérisée par le fait que** la concentration en matière sèche (CMS) est comprise entre 2,5 et 15 % en poids par rapport au poids total de la composition aérosol (jus + propulseur), de préférence comprise entre 3,5 et 10 % en poids.

**7.** Composition selon l'une quelconque des revendications précédentes, **caractérisée par le fait que** le débit en composition aérosol (DCA) est compris entre 500 et 800 mg/s, de préférence voisin de 600 mg/s.

**8.** Composition selon l'une quelconque des revendications précédentes, **caractérisée par le fait que** le rapport pondéral jus/propulseur est supérieur à 1, de préférence compris entre 1,2 et 3.

**9.** Composition cosmétique selon l'une quelconque des revendications précédentes, **caractérisée par le fait qu'**elle comprend en outre au moins un adjuvant cosmétique choisi parmi les agents conditionneurs de type esters, les agents anti-mousse, les agents hydratants, les agents émollients, les glycols, les plastifiants, les agents épaississants minéraux, les agents épaississants organiques, polymériques ou non, associatifs ou non, les filtres solaires hydrosolubles ou liposolubles, siliconés ou non siliconés, les colorants permanents ou temporaires, les parfums, les peptisants, les conservateurs, les céramides, et pseudo-céramides, les vitamines et les provitamines dont le panthénol, les protéines, les agents séquestrants, les agents solubilisants, les agents alcanisants, les agents acidifiants, les agents anti-corrosion, les agents réducteurs ou anti-oxydants, les agents oxydants, les charges minérales, les paillettes.

**10.** Composition cosmétique selon l'une quelconque des revendications précédentes, **caractérisée par le fait que** le milieu cosmétiquement acceptable est un milieu aqueux, alcoolique ou hydroalcoolique.

**11.** Procédé de traitement cosmétique, **caractérisé par le fait qu'**il comprend l'application d'une composition cosmétique selon l'une quelconque des revendications 1 à 10, sur les fibres kératiniques, telles que les cheveux.

**12.** Procédé de traitement cosmétique selon la revendication 11, **caractérisé par le fait que** l'application de ladite composition n'est pas suivie par un rinçage.

**13.** Utilisation d'une composition cosmétique selon l'une quelconque des revendications 1 à 10 pour la fixation des cheveux.

## Claims

**1.** Cosmetic composition for the treatment of keratinous fibres, in particular human keratinous fibres, such as the hair, in aerosol form, **characterized in that** it comprises, in a cosmetically acceptable medium,

- at least one polysaccharide of lambda-carrageenan type,
- carbon dioxide as propellent, and
- at least one additive chosen from a silicone, a fatty substance and a fixing polymer other than the polysaccharide of lambda-carrageenan type.

**2.** Cosmetic composition according to Claim 1, **characterized in that** the molecular weight (MW) of the polysaccharide of lambda-carrageenan type is of between 100 000 and 1 000 000, preferably between 250 000 and 800 000.

**3.** Cosmetic composition according to Claim 1 or 2, **characterized in that** the polysaccharide of lambda-carrageenan type is present in the cosmetic composition in an amount varying from 0.1 to 30% by weight, preferably in an amount varying from 0.2 to 20% by weight and more preferably still in an amount varying from 0.5 to 15% by weight, with respect to the total weight of the composition.

**4.** Composition according to any one of the preceding claims, **characterized in that** the propellent represents from 2 to 70%, preferably from 3 to 50%, by weight, with respect to the total weight of the combined compositions present in the aerosol device.

**5.** Composition according to any one of the preceding claims, **characterized in that** the aerosol device is appropriate for obtaining a dry matter throughput of greater than or equal to 20 mg/s, preferably of between 20 and 60 mg/s.

**6.** Composition according to any one of the preceding claims, **characterized in that** the concentration of dry matter (CDM) is between 2.5 and 15% by weight, with respect to the total weight of the aerosol composition (dispensable + propellent), preferably between 3.5 and 10% by weight.

**7.** Composition according to any one of the preceding claims, **characterized in that** the aerosol composition throughput (ACT) is between 500 and 800 mg/s, preferably in the vicinity of 600 mg/s.

**8.** Composition according to any one of the preceding claims, **characterized in that** the dispensable/propellent ratio by weight is greater than 1, preferably between 1.2 and 3.

**9.** Cosmetic composition according to any one of the preceding claims, **characterized in that** it additionally comprises at least one cosmetic adjuvant chosen from conditioning agents of ester type, antimousseing agents, moisturizing agents, emollients, glycols, plasticizers, inorganic thickening agents, polymeric or nonpolymeric and associative or non-associative organic thickening agents, water-soluble or fat-soluble sunscreens which may or may not be of silicone nature, permanent or temporary colourings, fragrances, peptizing agents, preservatives, ceramides and pseudoceramides, vitamins and provitamins, including panthenol, proteins, sequestering agents, solubilizing agents, basifying agents, acidifying agents, corrosion inhibitors, reducing agents or antioxidants, oxidizing agents, inorganic fillers or glitter.

**10.** Cosmetic composition according to any one of the preceding claims, **characterized in that** the cosmetically acceptable medium is an aqueous, alcoholic or aqueous/alcoholic medium.

**11.** Cosmetic treatment method, **characterized in that** it comprises the application of a cosmetic composition according to any one of Claims 1 to 10 to keratinous fibres, such as the hair.

**12.** Cosmetic treatment method according to Claim 11, **characterized in that** the application of the said composition is not followed by a rinsing.

**13.** Use of a cosmetic composition according to any one of Claims 1 to 10 for fixing the hair.

## Patentansprüche

**1.** Kosmetikzusammensetzung für die Behandlung von Keratinfasern, insbesondere menschlichen Keratinfasern wie das Haar, in Aerosolform, **dadurch gekennzeichnet, dass** sie

- mindestens ein Polysaccharid vom lambda-Carrageenan-Typ,
- Kohlendioxid als Treibmittel und
- mindestens einen Zusatzstoff aus der Reihe Silikon, Fettkörper und fixierendes Polymer, das sich von dem Polysaccharid des lambda-Carrageenan-Typs unterscheidet,

in einem kosmetisch unbedenklichen Medium umfasst.

**2.** Kosmetikzusammensetzung nach Anspruch 1, **dadurch gekennzeichnet, dass** das Molekulargewicht (mg) des Polysaccharids des lambda-Carrageenan-Typs zwischen 100.000 und 1.000.000, vorzugsweise zwischen 250.000 und 800.000, liegt.

**3.** Kosmetikzusammensetzung nach Anspruch 1 oder 2, **dadurch gekennzeichnet, dass** das Polysaccharid des lambda-Carrageenan-Typs in der Kosmetikzusammensetzung in einer Menge von 0,1 bis 30 Gew.-%, vorzugsweise in einer Menge von 0,2 bis 20 Gew.-% und noch stärker bevorzugt in einer Menge von 0,5 bis 15 Gew.-%, in Bezug auf das Gesamtgewicht der Zusammensetzung vorliegt.

**4.** Zusammensetzung nach einem der vorhergehenden Ansprüche, **dadurch gekennzeichnet, dass** das Treibmittel 2 bis 70 Gew.-%, vorzugsweise 3 bis 50 Gew.-%, in Bezug auf das Gesamtgewicht von allen in der Aerosolvorrichtung enthaltenden Zusammensetzungen ausmacht.

5. Zusammensetzung nach einem der vorhergehenden Ansprüche, **dadurch gekennzeichnet, dass** sich die Aerosolvorrichtung für das Erzielen eines Trockenmasseausstoßes von 20 mg/s oder darüber, vorzugsweise zwischen 20 und 60 mg/s, eignet.

6. Zusammensetzung nach einem der vorhergehenden Ansprüche, **dadurch gekennzeichnet, dass** die Trockenmassekonzentration (TMK) zwischen 2,5 und 15 Gew.-% in Bezug auf das Gesamtgewicht der Aerosolzusammensetzung (Flüssigkeit + Treibmittel), vorzugsweise zwischen 3,5 und 10 Gew.-%, beträgt.

7. Zusammensetzung nach einem der vorhergehenden Ansprüche, **dadurch gekennzeichnet, dass** der Aerosolzusammensetzungsausstoß (AZA) zwischen 500 und 800 mg/s, vorzugsweise ungefähr 600 mg/s, beträgt.

8. Zusammensetzung nach einem der vorhergehenden Ansprüche, **dadurch gekennzeichnet, dass** das Gewichtsverhältnis zwischen Flüssigkeit und Treibmittel über 1, vorzugsweise zwischen 1,2 und 3, beträgt.

9. Zusammensetzung nach einem der vorhergehenden Ansprüche, **dadurch gekennzeichnet, dass** sie weiterhin mindestens einen Kosmetikhilfsstoff, ausgewählt aus der Reihe Conditioner des Estertyps, Antischaummittel, Hydratisierungsmittel, zartmachende Mittel, Glykole, Weichmacher, mineralische Verdickungsmittel, gegebenenfalls Polymere und gegebenenfalls assoziative organische Verdickungsmittel, gegebenenfalls silikonhaltige wasserlösliche oder lipidlösliche Sonnenschutzfilter, Permanentfarbstoffe oder vorübergehende Farbstoffe, Peptisatoren, Konservierungsmittel, Ceramide und Pseudoceramide, Vitamine und Provitamine, darunter Panthenol, Proteine, Sequestriermittel, Lösungsmittel, Alkalinisierungsmittel, Ansäuerungsmittel, Korrosionsschutzmittel, Reduktionsmittel oder Antioxidationsmittel, Oxidationsmittel, mineralische Füllstoffe oder Metallflocken umfasst.

10. Zusammensetzung nach einem der vorhergehenden Ansprüche, **dadurch gekennzeichnet, dass** es sich bei dem kosmetisch unbedenklichen Medium um ein wässriges, alkoholisches oder wässrig-alkoholisches Medium handelt.

11. Kosmetikbehandlungsverfahren, **dadurch gekennzeichnet, dass** es die Applikation einer Kosmetikzusammensetzung nach einem der Ansprüche 1 bis 10 auf Keratinfasern, wie das Haar, umfasst.

12. Kosmetikbehandlungsverfahren nach Anspruch 11, **dadurch gekennzeichnet, dass** auf die Applikation der Zusammensetzung kein Spülen erfolgt.

13. Verwendung einer Kosmetikzusammensetzung nach einem der Ansprüche 1 bis 10 zum Fixieren des Haares.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1199064 A
- US 5070171 A
- US 5149765 A
- US 5093452 A
- US 5091493 A
- EP 0492657 A1
- EP 95238 A
- US 4185087 A
- EP 0530974 A
- EP 080976 A
- FR 2077143
- FR 2393573
- US 4131576 A
- FR 1222944
- DE 2330956
- US 2047398 A
- US 2723248 A
- US 2102113 A
- GB 839805 A
- FR 2350384
- FR 2357241
- FR 2198719
- US 4128631 A
- FR 1400366
- EP 0751162 A
- EP 0637600 A
- EP 0648485 A
- FR 2743297
- EP 0656021 A
- WO 9403510 A
- EP 0619111 A
- US 4874554 A
- US 4137180 A

### Littérature non-brevet citée dans la description

- **M.R. PORTER.** Handbook of Surfactants. Blackie & Son, 1991, 116-178